# EUROPÄISCHE PATENTANMELDUNG

(11) **EP 3 542 864 A1**
(43) Veröffentlichungstag der Anmeldung: **25.09.2019**
(21) Anmeldenummer: 19152543.5
(22) Anmeldetag: 18.01.2019
(51) Int. Cl.: A61Q 5/06, A61K 8/81, A61K 8/86, A61K 8/37, A61K 8/31, A61K 8/55, A61K 8/92, A61K 8/34, A61K 8/365

(54) **LOCKENBALSAM**

(30) Priorität: 21.03.2018 DE 102018204295
(71) Anmelder: Beiersdorf AG, 20253 Hamburg (DE)
(72) Erfinder: Reiter, Katharina, 21357 Barum (DE); Saß, Viola, 25488 Holm (DE)

(57) **Zusammenfassung**

Kosmetische Zubereitung für die Haare, insbesondere lockige und/oder gelockte Haare, die die Haare in einem Schritt pflegt und stylt. Die Zubereitung weist eine angenehme Haptik auf und liegt in Form einer Emulsion vor.

## Beschreibung

Die vorliegende Erfindung betrifft eine kosmetische Zubereitung für die Haare, insbesondere lockige und/oder gelockte Haare. Die Zubereitung pflegt und stylt die Haare in einem Schritt. Weiterhin zeichnet sich die Zubereitung dadurch aus, dass sie in Form einer Emulsion vorliegt und eine angenehme Haptik aufweist.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt das Haar eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft ist aus drei Schichten aufgebaut: Der zentrale Teil wird als Haarmark oder Medulla bezeichnet. Dieser Teil ist allerdings beim Menschen zurückgebildet und fehlt oft. An den zentralen Teil schließt sich die Haarrinde oder Cortex an. Diese Faserschicht besteht aus verhornten Faserzellen und bestimmt die Festigkeit und Elastizität des Haares. In dieser Schicht sind auch die Farbpigmente enthalten. Außen um die Faserschicht ist die Schuppenschicht oder Cuticula angeordnet. Diese Schicht ist mehrlagig, sehr dünn und durchsichtig.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf.

Es ist eine Vielzahl von Produkten bekannt, die speziell für die Anwendung auf dem Haar entwickelt wurden, Shampoos für die Haarwäsche, Haarspülungen für die Konditionierung nach der Haarwäsche, Festiger- und Stylingzubereitungen in verschiedenen Darreichungsformen, beispielsweise Gele, Lösungen, Mousses, Gelsprays für die Anwendung im nassen und/oder trockenen Haar, Haarsprays, Haargele, Haarwachse, Haaröle und andere mehr.

Haarcremes oder Frisiercremes sind an sich auch bekannt. In der Vergangenheit wurden derartige Produkte gerne von Männern benutzt, um der Frisur Halt zu geben und die Haare zu pflegen. Als Beispiel sei eine Haarcreme der Marke TABAC (Mintel Eintragsnummer 2855785) erwähnt.

Die Produktform eines Lockenbalsams ist eine relativ neue Produktform und wurde speziell für lockige und/oder gelockte Haare entwickelt. Zum einen sollen die Locken und der Erhalt einer lockigen Frisur unterstützt werden. Zum anderen sollen die Haare aber auch gepflegt werden.

Lockenbalsamzubereitungen sind bereits käuflich zu erwerben, wie die Produkte Curl Balm der Marke Nivea (Mintel Eintragsnummer 1872119) und Voluminizing Styling Curl Balm der Marke Maestro (Mintel Eintragsnummer 5325919) zeigen.

Es besteht aber weiterhin Bedarf, Verbesserungen bei Lockenbalsamzubereitungen herbei zu führen; so soll die pflegende Wirkung, insbesondere aber die Feuchtigkeits-spendende Wirkung, weiter verbessert werden, ohne dass diese Verbesserungen sich nachteilig auf die Stylingeigenschaften auswirken. Die Haptik des Lockenbalsams sollte auch verbessert werden, so dass der Eindruck von Klebrigkeit, Schmierigkeit oder Glitschigkeit vermieden wird. Die Zubereitung soll reichhaltig sein, gleichzeitig soll der Lockenbalsam sich mühelos auf dem Haar verteilen lassen und gut in die Haare einzuarbeiten sein. Geschehen die letztgenannten Schritte mit den Händen, so sollen die Hände anschließend weder klebrig, schmierig noch glitschig sein, sondern eher an der Pflege der Haare teilhaben, sich also auch gepflegt anfühlen.

Überraschenderweise wird dieses Bündel an Aufgaben gelöst durch eine kosmetische Zubereitung für die Haare, insbesondere lockige und/oder gelockte Haare, die in Form einer Emulsion vorliegt, enthaltend
- wenigstens eine Öl- und/oder Wachskomponente,
- Moisturizer, wobei in der Zubereitung wenigstens zwei Moisturizer enthalten sind,
- Emulgatoren, wobei in der Zubereitung wenigstens zwei Emulgatoren enthalten sind,
- wenigstens ein Stylingpolymer.

Es ist vorteilhaft, wenn die erfindungsgemäße Zubereitung auf humanem Kopfhaar zur Anwendung kommt. Besonders vorteilhaft ist es, wenn die erfindungsgemäße Zubereitung auf lockigem und/oder gelocktem Haar, das natürlichen Ursprungs sein kann, aber auch dauergewelltes Haar sein kann, zur Anwendung kommt.

Die erfindungsgemäße Zubereitung ist vorteilhaft eine wässrige Zubereitung und vorteilhaft enthält wenigstens 50 Gew.-% Wasser, bezogen auf das Gesamtgewicht der Zubereitung.

Die Ölphase (Fettphase) der erfindungsgemäßen Formulierungen umfasst ein oder mehrere Öl- und/oder Wachskomponenten.

Die Öl- und/oder Wachskomponenten können vorteilhaft polare Öle umfassen. Die Gruppe der polaren Öle umfasst insbesondere vorteilhaft die Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianssöl und andere mehr.

Die Öl- und/oder Wachskomponenten können ebenso vorteilhaft natürliche Wachse tierischen und pflanzlichen Ursprungs umfassen, wie beispielsweise Bienenwachs und andere Insektenwachse sowie Beeren-wachs, Sheabutter und/oder Lanolin (Wollwachs), wobei Sheabutter besonders vorteilhaft ist.

Die Öl- und/oder Wachskomponenten können auch vorteilhaft Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen umfassen. Solche Esteröle können dann besonders vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyl-oleat, Erucylerucat, Tridecylstearat, Tridecyltrimellitat, sowie synthetische, halbsynthetische und natürliche Gemische solcher Ester, wie z. B. Jojobaöl.

Auch besonders vorteilhaft enthält die erfindungsgemäße Zubereitung einen Gehalt an C12-15-Alkylbenzoat oder die Ölkomponente ist vorteilhaft C12-15-Alkylbenzoat.

Die Öl- und/oder Wachskomponenten können auch vorteilhaft Dialkylether und Dialkylcarbonate umfassen; vorteilhaft sind z. B. Dicaprylylether (Cetiol OE) und/oder Dicaprylylcarbonat, das beispielsweise unter der Handelsbezeichnung Cetiol CC bei der Fa. Cognis erhältlich ist.

Die Öl- und/oder Wachskomponenten können auch vorteilhaft Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C12-13-Alkyllactat, Di-C12-13-Alkyltartrat, Triisostearin, Di-pentaerythrityl Hexacaprylat/Hexacaprat, Propylenglykolmonoisostearat, Tricaprylin, Di-methylisosorbid.

Auch beliebige Abmischungen der genannten Öl- und Wachskomponenten sind vorteilhaft im Sinne der vorliegenden Erfindung einzusetzen.

Die Öl- und/oder Wachskomponenten können auch vorteilhaft unpolare Öle, die gewählt werden aus der Gruppe der verzweigten und unverzweigten Kohlenwasserstoffe und -wachse, insbesondere Mineralöl, Vaseline (Petrolatum), Paraffinöl, Squalan, Polyolefine, hydrogenierte Polyisobutene und Isohexadecan, umfassen. Das Mineralöl ist vorteilhaft ein Gelböl, das beispielsweise unter der Handelsbezeichnung Pionier 2076 bei der Firma H & R Chem Pharm erhältlich ist.

Der Gesamtgehalt der wenigstens einen Öl- und/oder Wachskomponente wird vorteilhaft aus dem Bereich von 0,1 bis 7,0 Gew. %, bevorzugt 1,0 bis 6,0 Gew. %, insbesondere bevorzugt 2,0 bis 5,0 Gew. %, bezogen auf das Gesamtgewicht der Zubereitung, gewählt.

Die erfindungsgemäße Zubereitung kann vorteilhaft Emollienzien enthalten. Unter Emollienzien werden Substanzen verstanden, die üblicherweise die Haut weich und geschmeidig machen. Derartige Substanzen sind auch vorteilhaft in der Haarpflege einzusetzen, denn auch die Haaroberfläche und/oder die Haarstruktur kann durch derartige Substanzen positiv beeinflusst werden. Das Haar fühlt sich weich und geschmeidiger an. Es gibt eine Vielzahl an Substanzen, die als Emollienzien wirken können. Im Sinne der vorliegenden Erfindung hat es sich als vorteilhaft erwiesen, C₁₂₋₁₅ Alkylbenzoat und/oder Lanolin Alkohol einzusetzen.

Wenn C₁₂₋₁₅ Alkylbenzoat als überwiegende oder alleinige Ölkomponente eingesetzt wird, hat diese Substanz die Funktion einer Ölkomponente und hat gleichzeitig auch die Wirkung eines Emolliens.

Wenn das wenigstens eine Emolliens zusätzlich zu oben genannten Öl- und/oder Wachskomponente in die erfindungsgemäße Zubereitung eingearbeitet wird, können auch Gehalte unter 0,1 Gew.-% vorteilhaft sein, insbesondere Gehalte von 0,01 bis 1,0 Gew.-%, bezogen auf das Gesamtgewicht der Zubereitung, vor.

Die erfindungsgemäße Zubereitung enthält Emulgatoren, wobei wenigstens zwei Emulgatoren enthalten sind. Besonders vorteilhaft weisen diese Emulgatoren verschiedene chemische Strukturen auf. Eine besonders vorteilhafte Gruppe von Emulgatoren sind Polysorbate, die Derivate des Sorbits sind. Polysorbate weisen weiterhin Ethylenoxidreste und meist langkettige Fettsäuren auf. Eine besonders bevorzugte Verbindung dieser Gruppe ist Polysorbat 60, das beispielsweise unter der Handelsbezeichnung SP Tween 60 MBAL-LQ-(MV) bei der Firma Croda erhältlich ist.

Eine weitere besonders vorteilhafte Gruppe an Emulgatoren ist die Gruppe der Monoester des Glycerins und meist langkettiger Fettsäuren, im folgenden Glycerylfettsäuren bezeichnet. Eine besonders bevorzugte Verbindung ist Glycerylstearate, die beispielsweise unter der Handelsbezeichnung Tegin M Pellets von der Firma Evonik erhältlich ist.

Erfindungsgemäß bevorzugt ist es, wenn die erfindungsgemäße Zubereitung eine Kombination an Emulgatoren aus wenigstens einem Polysorbate und wenigstens einer Glycerylfettsäure enthält. Es ist weiter bevorzugt, wenn das wenigstens eine Polysorbat zu der wenigstens einen Glycerylfettsäure im Gewichtsverhältnis von 1:0,1 bis 1:1, bevorzugt 1:0,2 bis 1:1, vorliegen.

Die erfindungsgemäßen Zubereitungen enthalten das wenigstens eine Polysorbat in einer Gesamtmenge von 0,5 bis 6,0 Gew.-%, bevorzugt 0,75 bis 5,0 Gew.-%, insbesondere bevorzugt 0,8 bis 4,0 Gew.-%, bezogen auf das Gesamtgewicht der Zubereitung.

Die erfindungsgemäßen Zubereitungen enthalten die wenigstens eine Glycerylfettsäure in einer Gesamtmenge von 0,5 bis 6,0 Gew.-%, bevorzugt 0,75 bis 5,0 Gew.-%, insbesondere bevorzugt 0,8 bis 4,0 Gew.-%, bezogen auf das Gesamtgewicht der Zubereitung.

Die erfindungsgemäße Zubereitung kann vorteilhaft Silikone enthalten. Silikone haben auch die chemische Bezeichnung Poly(organo)siloxane. Es handelt sich um eine Gruppe synthetischer Polymere, bei denen Siliziumatome über Sauerstoffatome verknüpft sind. Silikone bestehen aus einzelnen Siloxaneinheiten, die aus Siliciumatomen, verbunden über eine Sauerstoffbrücke, bestehen.

Die allgemeine Formel lautet RₙSiO_{(4-n)/2} (n=0, 1, 2, 3), d. h. eine Siloxaneinheit kann ein bis vier weitere Substituenten aufweisen.

Siloxaneinheiten können also mono-, di-, tri- und tetrafunktionell sein. In symbolischer Schreibweise stellt man dies durch folgende Buchstaben dar:
M (mono), M entspricht R₃SiO_{1/2},
D (di), D entspricht R₂SiO_{2/2},
T (tri), T entspricht RSiO_{3/2} und
Q (quatro), Q entspricht SiO_{4/2}.

Wie bei den organischen Polymeren basiert die Vielzahl der möglichen Verbindungen darauf, dass verschiedene Siloxaneinheiten im Molekül miteinander verknüpft werden können. Angelehnt an die Systematik der organischen Polymere kann man folgende Gruppen unterscheiden:
- Cyclische Polysiloxane sind ringförmig aus difunktionellen Siloxaneinheiten aufgebaut, Dₙ.
- Lineare Polysiloxane sind folgendermaßen aufgebaut MDₙM, bzw. R₃SiO[R₂SiO]ₙSiR₃, als Beispiel sei Polydimethylsiloxan genannt.
- Vernetzte Polysiloxane sind ketten- oder ringförmige Moleküle, die mit Hilfe von tri- und tetrafunktionellen Siloxaneinheiten zu ebenen oder dreidimensionalen Netzwerken verknüpft sind. Der Aufbau hochmolekularer Silikone erfolgt über Kettenbildung und Vernetzung.
- Verzweigte Polysiloxane weisen als verzweigende Elemente trifunktionelle oder tetrafunktionelle Siloxaneinheiten auf, MₙDₘTₙ. Die Verzweigungsstellen sind dabei entweder in eine Kette oder einen Ring eingebaut.

Cyclomethicone sind Silikonöle mit einer Ringstruktur. Sie sind praktisch wasserunlöslich. Der Name Cyclomethicone bezeichnet verschiedene Dimethylpolysiloxane, die bekanntesten sind Cyclopentasiloxane (10er-Ring, 5 Siliziumatome und 5 Sauerstoffatome), Cyclotrisiloxane (6er-Ring, 3 Siliziumatome und 3 Sauerstoffatome) und Cyclotetrasiloxane (8er-Ring, 4 Siliziumatome und 4 Sauerstoffatome). Es handelt sich um farblose, niedrigviskose und (schwach) flüchtige Flüssigkeiten. Cyclomethicone wirken spreitend. Sie werden häufig zusammen mit hochviskosen Silikonen eingesetzt. Sie können die Klebrigkeit verringern und können das Hautgefühl verbessern. In Haarpflegemitteln können sie als Konditionierer zur Verbesserung der Kämmbarkeit und des Glanzes der Haare wirken.

Dimethicone gehören zu der Gruppe der Silikonöle. Sie decken einen breiten Viskositätsbereich ab, von flüchtig bis hochviskos. Lineare Polydimethylsiloxane sind Polymere mit unterschiedlichen Molekülgrößen; sie sind praktisch wasserunlösliche, klare, farb- und geruchlose Flüssigkeiten. Der einfachste Vertreter, das Hexamethyldisiloxane, wird in Haarpflegemitteln zur Verbesserung der Kämmbarkeit, als Konditioniermittel und zur Erhöhung des Glanzes der Haare eingesetzt. Die flüssigen und flüchtigen Dimethicone sind Spreiter und Weichmacher, sie können zur Verbesserung der Nasskämmbarkeit der Haare eingesetzt werden.

Dimethiconol weist Hydroxylgruppen auf. Dadurch erlangt das Silikonmolekül polare Eigenschaften, wodurch eine gute Haftung am Haar erreicht wird. Dimethiconol ist hochviskos und wird deshalb oft in einer Mischung mit dem niedrigviskosen Cyclopentasiloxan angeboten. Diese Mischung kann zur Haarkonditionierung, beispielsweise in Haarspitzenfluids und als Antischaummittel, eingesetzt.

Die vorteilhaft einzusetzenden Silikonverbindungen können aus der Gruppe der Dimethicone ausgewählt werden. In die erfindungsgemäße Zubereitung werden bevorzugt Dimethicone eingearbeitet, die beispielsweise als Mirasil DM Series bei der Firma Bluestar Silicones erhältlich sind, oder als SF 96 Series von der Firma Momentive erhältlich oder als Xiameter PMX-200 Silicone Fluids von der Firma Dow Corning. Besonders bevorzugt ist ein Dimethicone, das charakterisiert ist durch eine Viskosität von 90 bis 110 mm²/s, gemessen bei 25°C; ein derartiges Dimethicone ist beispielsweise erhältlich als Mirasil DM 100 von der Firma Bluestar Silicones. Die erfindungsgemäße Zubereitung kann ein oder mehrere Dimethicone enthalten. Der Gesamtgehalt der Dimethicone in der erfindungsgemäßen Zubereitung beträgt 0,25 bis 6 Gew.-%, bevorzugt 0,5 bis 5 Gew.-%, besonders bevorzugt 1 bis 4 Gew.-%, bezogen auf das Gesamtgewicht der Zubereitung.

Auch Dimethiconole können vorteilhaft in die erfindungsgemäße Zubereitung eingearbeitet werden. Bevorzugt wird eine Mischung aus Dimethiconol und Cyclomethicone in die erfindungsgemäße Zubereitung eingearbeitet. Weiter bevorzugt ist es, wenn die Mischung eine Mischung aus 15 Gew.-% Dimethiconol und 85 Gew.-% Cyclopentasiloxane ist, wobei sich die Mengenangaben auf die Mischung beziehen. Eine derartige Mischung kann beispielsweise als Dow Corning® 1501 Fluid von der Firma Dow Corning bezogen werden.

Das Gemisch aus Dimethiconol und Cyclomethicone ist in der erfindungsgemäßen Zubereitung mit 0,25 bis 5 Gew.-%, bevorzugt 0,5 bis 4 Gew.-%, besonders bevorzugt 1-3 Gew.-% enthalten, bezogen auf das Gesamtgewicht der Zubereitung.

Die Viskosität der Silikone kann mit einem Glaskapillarviskosimeter nach der Methode Dow Corning Test Method CTM004, July20, 1970 bestimmt werden.

Als Moisturizer werden Stoffe oder Stoffgemische bezeichnet, welche kosmetischen Zubereitungen die Eigenschaft verleihen, nach dem Auftragen bzw. Verteilen auf der Haaroberfläche die Feuchtigkeitsabgabe zu reduzieren und/oder die Hydratation der Haaroberfläche bzw. der tiefer liegenden Schichten positiv zu beeinflussen. Vorteilhafte Moisturizer im Sinne der vorliegenden Erfindung sind beispielsweise Glycerin, Sorbitol, Butylenglykol, Propylenglykol, Milchsäure und/oder Lactate, insbesondere Natriumlactat, Biosaccaride Gum-1, Glycine Soja, Ethylhexyloxyglycerin, Pyrrolidoncarbonsäure und Harnstoff. Bevorzugt werden die Moisturizer ausgewählt aus der Gruppe Glycerin, Sorbitol, Butylenglykol, Propylenglykol, Milchsäure und/oder Lactate, insbesondere Natriumlactate. In der erfindungsgemäßen Zubereitung sind wenigstens zwei Moisturizer enthalten, wobei wenigstens einer aus der Gruppe der ausgewählten Verbindungen ausgewählt wird. Bevorzugt sind wenigstens zwei der Moisturizer aus der ausgewählten Gruppe ausgewählt. Weiter bevorzugt sind drei der Moisturizer aus der ausgewählten Gruppe ausgewählt, noch weiter bevorzugt sind vier der Moisturizer aus der Gruppe der ausgewählten Verbindungen ausgewählt. Am meisten bevorzugt sind in der erfindungsgemäßen Zubereitung Glycerin, Sorbitol, Butylenglycol und Propylenglycol enthalten.

Die wenigstens zwei Moisturizer liegen in der erfindungsgemäßen Zubereitung mit einem Gesamtgehalt von 2,25 bis 26 Gew.-%, bevorzugt 4,5 bis 19 Gew.-%, besonders bevorzugt 7 bis 17 Gew.-%, bezogen auf das Gesamtgewicht der Zubereitung vor.

Die erfindungsgemäße Zubereitung enthält wenigstens ein Haarstylingpolymer. Haarstylingpolymere sind in der Regel festigende Polymere, die einen Film auf dem Haar ausbilden können und so zur Fixierung der Frisur beitragen.

Haarstylingpolymere können verschiedene chemische Eigenschaften haben, beispielsweise sind anionische, kationische, amphotere und nichtionische Stylingpolymere bekannt. Im Sinne der vorliegenden Erfindung können Stylingpolymere aus allen genannten Gruppen ausgewählt werden.

Wenn das Stylingpolymer aus der Gruppe der nichtionischen Stylingpolymere ausgewählt wird, hat es sich als vorteilhaft erwiesen, das Stylingpolymer aus der Gruppe der nichtionischen Homo- und Copolymere enthaltend den Monomerbaustein Vinylpyrrolidon auszuwählen. So ist es insbesondere vorteilhaft, wenn das Stylingpolymer Polyvinylpyrrolidon (PVP) ist. PVP kann beispielsweise als Luviskol K 90 Pulver bei der Firma BASF erworben werden. Ebenso insbesondere vorteilhaft ist es, wenn das Stylingpolymer ein VP/VA Copolymer ist, das beispielsweise als Luviskol VA 64 W von der Firma BASF bezogen werden kann.

Wenn das Stylingpolymer aus der Gruppe der anionischen Polymere ausgewählt wird, hat es sich als vorteilhaft erwiesen, ein Acrylates/Hydroxyesters Acrylates Copolymer auszuwählen. Ein derartiges Polymer ist beispielsweise als Acudyne 180 von der Firma Dow Chemical erhältlich.

Wenn das Stylingpolymer aus der Gruppe der kationischen Polymere ausgewählt wird, hat es sich als vorteilhaft erwiesen, Polyquaternium-4 auszuwählen. Ein derartiges Polymer ist beispielsweise als Celquat L-200 von der Firma Akzo Nobel erhältlich.

Wenn das Stylingpolymer aus der Gruppe der amphoteren Polymere ausgewählt wird, hat es sich als vorteilhaft erwiesen, ein Octylacrylamide/Acrylates/Butylaminoethyl Methacrylate Copolymer auszuwählen. Ein derartiges Polymer ist beispielsweise als Amphomer von der Firma Akzo Nobel erhältlich.

Das wenigstens ein Haarstylingpolymer ist mit einem Gesamtgehalt von 0,05 bis
8,0 Gew. %, bevorzugt 0,5 bis 6,0 Gew. %, besonders bevorzugt 1,0 bis 4,0 Gew. %, bezogen auf das Gesamtgewicht der Zubereitung, in der erfindungsgemäßen Zubereitung enthalten.

Die erfindungsgemäße Zubereitung kann vorteilhaft wenigstens einen Verdicker enthalten. Unter Verdickern werden Makromoleküle verstanden, die einen weitgehend linearen, teilweise auch vernetzten Aufbau haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche oder wasser-quellbare natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen und wasserquellbaren Polymere stellen eine große Gruppe chemisch sehr unterschiedlicher natürlicher und synthetischer Polymere dar, deren gemeinsames Merkmal ihre Löslichkeit oder Quellbarkeit in Wasser bzw. wässrigen Medien ist. Voraussetzung dafür ist, dass diese Polymere über eine für die Wasserlöslichkeit oder Wasserquellbarkeit ausreichende Anzahl an hydrophilen Gruppen besitzen und nicht zu stark vernetzt sind. Die hydrophilen Gruppen können nichtionischer, anionischer oder kationischer Natur sein.

Die Gruppe der polymeren Strukturanten, die vielfach in der Kosmetik, aber auch in der Dermatologie, zum Einsatz kommen, lässt sich wie folgt einteilen in:
- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide, Co-Polymere und hydrophob modifizierte Polymere,
- anorganische Verbindungen, wie z. B. Polykieselsäuren, Tonmineralien wie Montmorillonite, Zeolithe, Kieselsäuren.

Der wenigstens eine Verdicker kann vorteilhaft aus der Gruppe Polyacrylate gewählt werden, also synthetische Polymere, die als Monomere wenigstens eine Verbindung aus der Gruppe der (Meth-)Acrylsäure, der Salze der (Meth-)Acrylsäure und der Derivate der (Meth-) Acrylsäure aufweist. Derartige Verdicker können besonders vorteilhaft aus den folgenden Substanzen gewählt werden: lineare Polyacrylate, welche allgemein als Carbomere bekannt sind (zum Beispiel Carbopol® Ultrez 10 Polymer, Carbopol® Ultrez 30 Polymer oder Carbopol® 980 Polymer von der Gesellschaft Lubrizol) sowie Acrylates/C10-30 Alkyl Acrylate Crosspolymer (zum Beispiel Carbopol® Ultrez 20 Polymer, Carbopol® Ultrez 21 Polymer, Carbopol® ETD 2020 Polymer, Carbopol® 1382 Polymer, Carbopol® 5984 Polymer von der Gesellschaft Lubrizol).

Weiterhin einzusetzende Acrylatverdicker sind die Substanzen, die von der Gesellschaft Lubrizol unter der Bezeichnung Carbopol® Aqua SF-1 Polymer (INCI-Bezeichnung: Acrylates Copolymer) oder Carbopol® Aqua SF-2 Polymer (INCI-Bezeichnung:Acrylates Crosspolymer-4) oder ACECare von der Fa. KCl oder Worlee Gel 230 der Fa. Worlee (beides Verbindungen mit der INCI-Bezeichnung: Acrylates Copolymer) verkauft werden.

Weitere Vertreter dieser Polyacrylatklasse werden in DE 10 2010 022 063 beschrieben. Substanzen dieser Polymerklasse stellen quervernetzte Acrylat-Copolymere dar, welche folgende Strukturkomponenten enthalten,
I. saure Vinylmonomere und/oder ihre Salze (wie zum Beispiel Acrylsäure oder Methacrylsäure),
II. nichtionische Vinylmonomere (zum Beispiel C₁ bis C₅ Alkylester einer Acrylsäure),
III. ein oder mehrere quervernetzende Monomere und
IV. Monomere, die eine ungesättigte Endgruppe und einen Polyoxyalkylenteil enthalten.

Ganz besonders bevorzugt wird der wenigstens eine Verdicker ausgewählt aus Acrylates/C10-30 Alkyl Acrylate Crosspolymeren, beispielsweise erhältlich als Carbopol® Ultrez 20 Polymer, Carbopol® Ultrez 21 Polymer, Carbopol® ETD 2020 Polymer, Carbopol® 1382 Polymer, Carbopol® 5984 Polymer von der Gesellschaft Lubrizol. Der am meisten bevorzugte Verdicker ist ein Acrylates/C10-30 Alkyl Acrylate Crosspolymere, das unter der Handelsbezeichnung Carbopol® Ultrez 21 Polymer von der Firma Lubrizol erhältlich ist.

Die Gesamtmenge an wenigstens einem Verdicker in der erfindungsgemäßen Zubereitung wird vorteilhaft aus dem Bereich von 0,01 bis 2,0 Gew.-%, bevorzugt 0,05 bis 1 Gew.-%, insbesondere bevorzugt 0,075 bis 0,5 Gew.-% gewählt, bezogen auf das Gesamtgewicht der Zubereitung.

Neben den vorgenannten Verdickern können weitere Substanzen zum Einsatz kommen, die eine verdickende Wirkung haben oder die Wirkung der oben genannten Verdicker verstärken und oder unterstützen.

Eine derartige Substanz ist Polyethylenglycol. Polyethylenglycol abgekürzt PEG ist in Abhängigkeit von der Kettenlänge ein flüssiges oder festes Polymer, das wasserlöslich ist. Die Wiederholeinheit, Ethylenoxid oder EO, des linear aufgebauten Polymers ist (-CH2-CH2-O-). Die Abkürzung PEG 90M steht für ein Polymer aus 90.000 Ethylenoxideinheiten. In kosmetischen Zubereitungen können Polyethylenglycole Emulsionen stabilisieren und verdickend wirken.

Vorteilhaft ist es, wenn in der erfindungsgemäßen Zubereitung PEG 90M enthalten ist. Dieses hat zusätzlich Einfluss auf die Sensorik. Die Sensorik entsprechender Zubereitungen ist reichhaltig und geschmeidig. Besonders vorteilhaft liegt PEG 90M im Gemisch mit Silica vor, ganz besonders vorteilhaft in einem Gemisch aus 95 bis 97 Gew.-% PEG 90M und 2 bis 3 Gew.-% Silica, wobei sich diese Gewichtsangaben auf das Gemisch beziehen.

Die Menge an PEG 90M in der erfindungsgemäßen Zubereitung wird vorteilhaft aus dem Bereich 0,01 bis 1,0 Gew.-%, bevorzugt 0,02 bis 0,8 Gew.-%, besonders bevorzugt 0,04 bis 0,75 Gew.-%, bezogen auf das Gesamtgewicht der Zubereitung, ausgewählt.

Weitere Substanzen, die Einfluss auf die Viskosität einer Zubereitung haben, sind Fettalkohole. Fettalkohole sind die Alkohole höherer Fettsäuren. Vorteilhaft im Sinne der vorliegenden Erfindung ist es, wenn Cetylalkohol und/oder Stearylalkohol zum Einsatz kommen. Die Mischung aus Cetylalkohol und Stearylalkohol wird häufig Cetearylalkohol bezeichnet.

Der wenigstens eine Fettalkohol liegt in der erfindungsgemäßen Zubereitung vorteilhaft mit einem Gesamtgehalt von 0,5 bis 6,0 Gew.-%, bevorzugt 0,75 bis 5,0 Gew.-%, besonders bevorzugt 0,8 bis 4,0 Gew.-% vor.

Die erfindungsgemäße Zubereitung kann vorteilhaft zusätzliche Wirkstoffe enthalten, die aus der Gruppe der hydrolysierten Proteine ausgewählt werden können.

Besonders vorteilhaft ist es, wenn die erfindungsgemäße Zubereitung ein oder mehrere Proteinhydrolysat(e) und/oder Derivate von Proteinhydrolysaten enthält.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemaess einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Ganz besonders vorteilhaft ist es, wenn die erfindungsgemäße Zubereitung hydrolysiertes Milchprotein und/oder hydrolysierte Seidenprotein enthält. Derartige Hydrolysate sind beispielsweise unter den Handelsbezeichnungen Crosilk Protein Complex NSP-LQ-(WD) und/oder Hydrolactin 2500 bei der Firma Croda erhältlich.

Das oder die Proteinhydrolysate sind vorteilhaft mit einem Gesamtgehalt von 0,001 bis 1 Gew.-%, bevorzugt 0,005 bis 0,8 Gew.-%, bezogen auf das Gesamtgewicht der Zubereitung, in der erfindungsgemäßen Zubereitung enthalten.

Im Sinne der vorliegenden Erfindung ist es vorteilhaft, wenn die erfindungsgemäße Zubereitung konserviert ist. Dazu können alle Konservierungsstoffe verwendet werden, die gemäß Kosmetikverordnung verwendet werden dürfen. Bevorzugt ist jedoch eine Konservierung mit Parabenen und/oder Phenoxyethanol.

Parabene im Sinne der vorliegenden Erfindung haben folgende allgemeine Strukturformel:

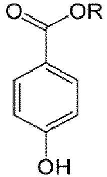

R bedeutet Alkylseitenkette, die aus einer unverzweigten oder verzweigten Kette von 1 bis 5 Kohlenstoffatomen oder aus aromatischen Resten bestehen kann. Bekannte Parabene sind Methyl-, Ethyl-, Propyl- Butylparaben, ebenso wie Isopropyl-, Isobutyl-, Pentyl- und Phenylparaben. Benzylparaben ist in kosmetischen Mitteln als Konservierungsmittel nicht zugelassen. Bevorzugt werden die Parabene ausgewählt aus der Gruppe Methyl- und/oder Ethylparaben.

Wenn Parabene zur Konservierung der erfindungsgemäßen Zubereitung eingesetzt werden, dann liegt das wenigstens eine Paraben vorteilhaft mit einem Gesamtgehalt von 0,01 bis 1,0 Gew.-%, bevorzugt 0,02 bis 0,75 Gew.-%, besonders bevorzugt 0,05 bis 0,5 Gew.-%, bezogen auf Gesamtgewicht der Zubereitung, vor.

Ebenso vorteilhaft ist eine Konservierung mit Phenoxyethanol. Wenn Phenoxyethanol zur Konservierung der erfindungsgemäßen Zubereitung eingesetzt wird, liegt Phenoxyethanol mit einem Gehalt von 0,1 bis 1,0 Gew.-%, bevorzugt 0,05 bis 0,8 Gew.-%, bezogen auf das Gesamtgewicht der Zubereitung, vor.

Der pH-Wert der erfindungsgemäßen Zubereitung kann durch alle Substanzen eingestellt werden, die üblicherweise in der Kosmetik eingesetzt werden, um den pH-Wert der erfindungsgemäßen Zubereitungen einzustellen und stabil zu halten. Vorteilhaft können diese Substanzen ausgewählt werden aus der Gruppe Zitronensäure und Milchsäure und/oder aus der Gruppe Aminomethylpropanol, Natronlauge, Kalilauge und Triethanolamin.

Optional können, wenn erforderlich, die in der Kosmetik üblichen Zusatz- und Hilfsstoffe in die Zubereitungen eingearbeitet werden, wie z.B. Vitamine, Mineralstoffe, Pflanzenextrakte, desodorierend wirkende Substanzen, Antitranspirantien, Insektenrepellentien, Farbstoffe, Pigmente mit färbender Wirkung, Geschmacksstoffe, Vergällungsmittel, Parfums, Antioxidantien, UV-Filtersubstanzen, Sensorikadditive oder andere übliche Bestandteile einer kosmetischen Formulierung wie Elektrolyte oder organische Lösungsmittel, mit der Maßgabe, dass der Einsatz derartiger Stoffe der Lösung der vorstehenden Aufgabe nicht zuwiderläuft.

Die erfindungsgemäße Zubereitung wird in Verpackungen zur Verfügung gestellt, die in der kosmetischen Industrie üblicherweise zum Einsatz kommen, wie Spender, Tiegel, Pumpspender und andere mehr.

Um zu belegen, dass das erfindungsgemäße Produkt, die Aufgaben der vorliegenden Erfindung löst, nämlich insbesondere die Haare mit mehr Feuchtigkeit zu versorgen, ohne dabei die Frisur (Locken) zu beeinträchtigen, wurde eine beispielhafte, erfindungsgemäße Zubereitung (ein Produkt gemäß Beispielrezeptur 1) in einem Home-In-Use-Test untersucht. 84 weibliche Probandinnen erhielten das genannte Produkt und sollten es für 10 Tage anwenden. In dieser Zeit durfte kein anderer Leave-in-Balsam verwendet werden. Es wurde weiterhin ein Fragebogen verteilt, in dem verschiedene Produkteigenschaften und Haareigenschaften nach der Anwendung des Produktes abgefragt wurden. 79 Fragebogen wurden abgegeben und konnten ausgewertet werden.

Die Aussagen, die sich auf den Erhalt der Locken bezogen lauteten:
- Das Produkt definiert meine Locken, 82 % der Probandinnen bestätigen diese Aussage;
- das Produkt definiert meine Locken perfekt, 82 % der Probandinnen bestätigen diese Aussage;
- mein Haar ist harmonisch gestylt, 85 % der Probandinnen bestätigen diese Aussage;
- meine Locken sind langanhaltend definiert, 78 % der Probandinnen bestätigen diese Aussage;
- das Produkt zähmt meine Locken, 84 % der Probandinnen bestätigen diese Aussage.

Es wird deutlich, dass das zu bewertende Produkt in Bezug auf die Locken und deren Erhalt sehr positiv beurteilt wird.

Die Aussagen, die sich auf die Versorgung der Haare mit Feuchtigkeit bezogen, lauteten folgendermaßen:
- Das Produkt versorgt mein Haar mit Feuchtigkeit, 87 % der Probandinnen bestätigen diese Aussage;
- Mein Haar wurde sichtbar mit Feuchtigkeit versorgt, 87 % der Probandinnen bestätigen diese Aussage;
- Das Produkt bietet merkbare, intensive Feuchtigkeit, 87 % der Probandinnen bestätigen diese Aussage;
- Das Produkt bietet die Feuchtigkeit, die mein Haar braucht, 94 % der Probandinnen bestätigen diese Aussage.

Auch hier wird deutlich, dass die Versorgung des Haares mit Feuchtigkeit von den Probandinnen wahrgenommen wird und sehr positiv beurteilt wird.

Diese Studie kann als deutliches Indiz gewertet werden, dass die Aspekte der Problemstellung in Bezug auf Lockenerhalt und Feuchtigkeitsversorgung von gelocktem und/oder lockigem Haar von der erfindungsgemäßen Zubereitung adressiert wurde und durch die sehr positive Bewertung der Probandinnen kann die Aufgabe als gelöst betrachtet werden.

### Beispiele:

Die Angaben sind in Gew.-% und beziehen sich auf Aktivgehalte.

| | **INCI** | [Gew .-%] | [Gew. -%] | [Gew. -%] | [Gew. -%] | [Gew. -%] |
|---|---|---|---|---|---|---|
| Fettphase (A) 75°C | C₁₂₋₁₅ Alkyl Benzoate | 1 | 1 | 4 | 1 | 1 |
| | Paraffinum Liquidum | 5 | | | 2 | 2 |
| | Methylparaben | 0,1 | | | 0,1 | 0,1 |
| | Cetearyl Alcohol | 2 | 2 | 2 | 2 | 2 |
| | Polysorbate 60 | 2 | 2 | 2 | 2 | 2 |
| | Glyceryl Stearate | 1 | 1 | 1 | 1 | 1 |
| | Phenoxyethanol | 0.6 | 0.9 | 0.9 | 0.6 | 0.6 |
| | Lanolin Alcohol | 0.5 | 0.5 | 0.5 | 0.5 | 0.5 |
| | Butyrospermum Parkii Butter | | 3 | | 2 | 2 |
| | | | | | | |
| Silikonöle (B) | Dimethicone | 2.5 | 2.3 | 2 | 2.5 | 2.5 |
| | Gemisch aus 85% Cyclomethicone + 15% Dimethiconol | 1.5 | 2.5 | 2 | 1.5 | 1.5 |
| | | | | | | |
| Wasserphase (C) 75°C | Aqua | Add 56,3 | Add 58,1 | Add 58,1 | Add 57,3 | Add 57,3 |
| | Acrylates/C1 0-30 Alkyl Acrylate Crosspolymer | 0.1 | 0.1 | 0.1 | 0.1 | 0.1 |
| | Hydrolyzed Milk Protein | 0.4 | 0.4 | 0.4 | 0.4 | 0.4 |
| | Hydrolyzed Silk | 0.45 | 0.45 | 0.45 | 0.45 | 0.45 |
| | Glycerin | 4 | 4 | 4 | 4 | 4 |
| | Sorbitol | 3.5 | 3.5 | 3.5 | 3.5 | 3.5 |
| | Butylene Glycol | 3 | 3 | 3 | 3 | 3 |
| | | | | | | |
| Extraphase 1 (D) | Pro pylene Glycol | 2 | 2 | 2 | 2 | 2 |
| | PEG-90 M | 0.1 | 0.1 | 0.1 | 0.1 | 0.1 |
| | | | | | | |
| Extraphase 2 (F) | PVP | 4 | 2 | | | |
| | PVP/VA Copolymer | | 1.5 | | | |
| | Octylacrylamide/Acrylates/Butyl-aminoethyl Methacrylate Copolymer | | | 3 | | |
| | Polyquaternium-4 | | | | 2.5 | |
| | Acrylates/Hydroxyesters Acrylates Copolymer | | | | | 2 |
| | Aqua | Add 25 | Add 25 | Add 25 | Add 25 | Add 25 |
| | Aminomethyl propanol | | | Adj. | | Adj. |
| | Sodium Hydroxide | 0.135 | 0.135 | | 0.135 | |
| | | | | | | |
| Parfümphase (G) | Parfum | 0.4 | 0.4 | 0.4 | 0.4 | 0.4 |

Komponenten der Fettphase (A) vorlegen, mischen und auf etwa 75°C erwärmen;
Komponenten der Wasserphase (C) mischen, auf etwa 75°C erwärmen;
Phase (A) und Phase (C) vereinigen und homogenisieren;
Zugabe von Phase (B) zu der vereinigten Phase (A) + (C), homogenisieren;
Zugabe von Phase (D) und Phase (F) zu der vereinigten Phase (A) + (B) + (C);
Abkühlen auf eine Temperatur < 35°C;
Zugabe der Phase (G) zu der vereinigten Phase (A) + (B) + (C) + (D) + (F), homogenisieren.

## Patentansprüche

1. Kosmetische Zubereitung für die Haare, insbesondere lockige und/oder gelockte Haare, in Form einer Emulsion vorliegend und enthaltend
- wenigstens eine Öl- und/oder Wachskomponente,
- Moisturizer, wobei in der Zubereitung wenigstens zwei Moisturizer enthalten sind,
- Emulgatoren, wobei in der Zubereitung wenigstens zwei Emulgatoren enthalten sind,
- wenigstens ein Stylingpolymer.

2. Zubereitung nach Anspruch 1 **dadurch gekennzeichnet, dass** die wenigstens eine Öl- und/oder Wachskomponente wenigstens ein polares Öl umfasst,
- ausgewählt aus der Gruppe der Lecithine und der Fettsäuretriglyceride, insbesondere Triglycerinester gesättigter und/oder ungesättigter, verzweigter und/oder unverzweigter Alkancarbonsäuren einer Kettenlänge von 8 bis 24, insbesondere 12 bis 18 C-Atomen
- ausgewählt aus natürlichen Wachsen tierischen und pflanzlichen Ursprungs, bevorzugt Shea butter,
- ausgewählt aus Estern aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen, insbesondere C₁₂-₁₅-Alkylbenzoat,
- ausgewählt aus Dialkylethern und Dialkylcarbonaten und
- Mischungen davon.

3. Zubereitung nach Anspruch 1 und/oder 2 **dadurch gekennzeichnet, dass** die wenigstens eine Öl- und/oder Wachskomponente wenigstens ein unpolares Öl umfasst, ausgewählt aus der Gruppe der verzweigten und unverzweigten Kohlenwasserstoffe und - wachse, Vaseline (Petrolatum), Paraffinöl, Squalan, Polyolefine, hydrogenierte Polyisobutene und/oder Isohexadecan, bevorzugt Mineralöl.

4. Zubereitung nach wenigstens einem der vorstehenden Ansprüche **dadurch gekennzeichnet, dass** das die wenigstens eine Öl- und/oder Wachskomponente mit einem Gesamtgehalt von 0,1 bis 7,0 Gew.-%, bevorzugt 1,0 bis 6,0 Gew.-%, insbesondere bevorzugt 2,0 bis 5,0 Gew.-%, bezogen auf das Gesamtgewicht der Zubereitung, enthalten ist.

5. Zubereitung nach wenigstens einem der vorstehenden Ansprüche **dadurch gekennzeichnet, dass** wenigstens ein Emulgator der wenigstens zwei Emulgatoren ausgewählt wird aus der Gruppe Polysorbate und/oder Glycerylfettsäuren.

6. Zubereitung nach wenigstens einem der vorstehenden Ansprüche **dadurch gekennzeichnet, dass** als Emulgatoren wenigstens ein Polysorbat und wenigstens eine Glycerylfettsäure enthalten ist.

7. Zubereitung nach wenigstens einem der vorstehenden Ansprüche **dadurch gekennzeichnet, dass** als Emulgatoren Polysorbat 60 und Glycerylstearat enthalten sind.

8. Zubereitung nach wenigstens einem der vorstehenden Ansprüche **dadurch gekennzeichnet, dass** das wenigstens eine Polysorsobat mit einem Gesamtgehalt von 0,5 bis 6,0 Gew.-%, bevorzugt 0,75 bis 5,0 Gew.-%, insbesondere bevorzugt 0,8 bis 4,0 Gew.-%, bezogen auf das Gesamtgewicht der Zubereitung, enthalten ist.

9. Zubereitung nach wenigstens einem der vorstehenden Ansprüche **dadurch gekennzeichnet, dass** die wenigstens eine Glycerylfettsäure mit einem Gesamtgehalt 0,5 bis 6,0 Gew.-%, bevorzugt 0,75 bis 5,0 Gew.-%, insbesondere bevorzugt 0,8 bis 4,0 Gew.-%, bezogen auf das Gesamtgewicht der Zubereitung, enthalten ist.

10. Zubereitung nach wenigstens einem der vorstehenden Ansprüche **dadurch gekennzeichnet, dass** das wenigstens eine Polysorbat und die wenigstens eine Glycerylfettsäure im Gewichtsverhältnis von Gewichtsverhältnis von 1:0,1 bis 1:1, bevorzugt 1:0,2 bis 1:1, vorliegen.

11. Zubereitung nach wenigstens einem der vorstehenden Ansprüche **dadurch gekennzeichnet, dass** zusätzlich Silikone enthalten sind, bevorzugt ein oder mehrere Dimethicone und ein oder mehrere Dimethiconole, weiter bevorzugt ein oder mehrere Dimethicone und eine Mischung aus Dimethiconol und Cyclomethicone.

12. Zubereitung nach wenigstens einem der vorstehenden Ansprüche **dadurch gekennzeichnet, dass** das oder die Dimethicone mit einem Gesamtgehalt von 0,25 bis 6 Gew.-%, bevorzugt 0,5 bis 5 Gew.-%, besonders bevorzugt 1 bis 4 Gew.-%, bezogen auf das Gesamtgewicht der Zubereitung, enthalten sind.

13. Zubereitung nach wenigstens einem der vorstehenden Ansprüche **dadurch gekennzeichnet, dass** die Mischung aus Dimethiconol und Cyclomethicone eine Mischung aus 15 Gew.-% Dimethiconol und 85 Gew.-% Cyclomethicon ist, wobei sich die Mengenangaben auf die Mischung beziehen.

14. Zubereitung nach wenigstens einem der vorstehenden Ansprüche **dadurch gekennzeichnet, dass** die Mischung aus Dimethiconol und Cyclomethicone in der erfindungsgemäßen Zubereitung mit 0,25 bis 5 Gew.-%, bevorzugt 0,5 bis 4 Gew.-%, besonders bevorzugt 1-3 Gew.-%, bezogen auf das Gesamtgewicht der Zubereitung, enthalten ist.

15. Zubereitung nach wenigstens einem der vorstehenden Ansprüche **dadurch gekennzeichnet, dass** wenigstens ein Moisturizer der wenigstens zwei Moisturizer ausgewählt wird aus der Gruppe Glycerin, Sorbitol, Butylenglykol, Propylenglykol, Milchsäure und/oder Lactate, insbesondere Natriumlactate.

16. Zubereitung nach wenigstens einem der vorstehenden Ansprüche **dadurch gekennzeichnet, dass** wenigstens zwei Moisturizer ausgewählt werden aus der Gruppe Glycerin, Sorbitol, Butylenglykol, Propylenglykol, Milchsäure und/oder Lactate, insbesondere Natriumlactate, bevorzugt das wenigstens drei Moisturizer ausgewählt werden aus der Gruppe Glycerin, Sorbitol, Butylenglykol, Propylenglykol, Milchsäure und/oder Lactate, insbesondere Natriumlactate.

17. Zubereitung nach wenigstens einem der vorstehenden Ansprüche **dadurch gekennzeichnet, dass** als Moisturizer Glycerin, Sorbitol, Butylenglycol und Propylenglycol enthalten sind.

18. Zubereitung nach wenigstens einem der vorstehenden Ansprüche **dadurch gekennzeichnet, dass** die wenigstens zwei Moisturizer mit einem Gesamtgehalt von 2,25 bis 26 Gew.-%, bevorzugt 4,5 bis 19 Gew.-%, besonders bevorzugt 7 bis 17 Gew.-%, bezogen auf das Gesamtgewicht der Zubereitung, enthalten sind.

19. Zubereitung nach wenigstens einem der vorstehenden Ansprüche **dadurch gekennzeichnet, dass** das wenigstens eine Haarstylingpolymer mit einem Gesamtgehalt von 0,05 bis 8,0 Gew.-%, bevorzugt 0,5 bis 6,0 Gew.-%, besonders bevorzugt 1,0 bis 4,0 Gew.-%, bezogen auf das Gesamtgewicht der Zubereitung, enthalten ist.

20. Zubereitung nach wenigstens einem der vorstehenden Ansprüche **dadurch gekennzeichnet, dass** zusätzlich wenigstens ein Verdicker enthalten ist, bevorzugt ein Verdicker aus der Gruppe der Polyacrylate, insbesondere bevorzugt Acrylates/C10-30 Alkyl Acrylate Crosspolymer.

21. Zubereitung nach wenigstens einem der vorstehenden Ansprüche **dadurch gekennzeichnet, dass** der wenigstens eine Verdicker mit einem Gesamtgehalt von 0,01 bis 2,0 Gew.-%, bevorzugt 0,02 bis 1 Gew.-%, insbesondere bevorzugt 0,03 bis 0,5 Gew.-%, bezogen auf das Gesamtgewicht der Zubereitung, enthalten ist.

22. Zubereitung nach wenigstens einem der vorstehenden Ansprüche **dadurch gekennzeichnet, dass** zusätzlich ein oder mehrere Konservierungsstoff(e), bevorzugt ausgewählt aus Parabenen und/oder Phenoxyethanol, enthalten sind.

23. Zubereitung nach wenigstens einem der vorstehenden Ansprüche **dadurch gekennzeichnet, dass** das wenigstens eine Paraben mit einem Gesamtgehalt von 0,01 bis 1,0 Gew.-%, bevorzugt 0,02 bis 0,75 Gew.-%, besonders bevorzugt 0,05 bis 0,5 Gew.-%, bezogen auf Gesamtgewicht der Zubereitung, enthalten ist.

24. Zubereitung nach wenigstens einem der vorstehenden Ansprüche **dadurch gekennzeichnet, dass** Phenoxyethanol mit einem Gehalt von 0,1 bis 1,0 Gew.-%, bevorzugt 0,05 bis 0,8 Gew.-%, bezogen auf das Gesamtgewicht der Zubereitung, enthalten ist.
